# FASCICULE DE BREVET EUROPEEN

(11) **EP 0 879 589 B1**
(45) Date de publication et mention de la délivrance du brevet: **03.04.2002**
(21) Numéro de dépôt: 98400450.7
(22) Date de dépôt: 25.02.1998
(51) Int. Cl.: A61K 7/00, A61K 7/48

(54) **Nanoémulsion à base de lipides amphiphiles non-ioniques et de silicones aminées et utilisations**
Aus nicht-ionischen amphiphilen Fettstoffen und Aminosilikonen bestehende Nanoemulsionen und deren Verwendung.
Nanoemulsions based on non-ionic amphiphilic lipids aminated silicones and their use

(30) Priorité: 18.03.1997 FR 9703283
(43) Date de publication de la demande: 25.11.1998
(73) Titulaire: L'OREAL, 75008 Paris (FR)
(72) Inventeur: Cervantes, Frédéric, 75017 Paris (FR); Cazin, Bénédicte, 92110 Clichy (FR); Simonnet, Jean-Thierry, 75011 Paris (FR)
(74) Mandataire: Le Blainvaux Bellegarde, Françoise

(56) Documents cités:
- EP-A- 0 514 934
- EP-A- 0 529 883
- EP-A- 0 532 256
- WO-A-94/22420
- WO-A-95/15742
- FR-A- 2 718 019
- US-A- 4 529 586
- US-A- 5 098 745

## Description

La présente invention a trait à une émulsion huile-dans-eau dont les globules d'huile ont une taille moyenne inférieure à 150 nm et comprenant au moins une huile, au moins une silicone aminée et une phase lipidique amphiphile à base de lipides amphiphiles non-ioniques liquides à une température ambiante inférieure à 45°C ainsi qu'à leur utilisation en application topique notamment en cosmétique et en dermopharmacie.

Selon l'invention, on appelle nanoémulsions des émulsions dont les globules d'huile ont une taille moyenne inférieure à 150 nm (nanomètres).

Les émulsions huile-dans-eau sont bien connues dans le domaine de la cosmétique et de la dermopharmacie notamment pour la préparation de produits cosmétiques tels que des lotions, des toniques, des sérums, des eaux de toilette.

Cependant, la présence de concentrations importantes d'huiles végétales, animales ou minérales dans des compositions rend leur formulation difficile. En effet, les compositions sont généralement instables au stockage et les propriétés cosmétiques sont insuffisantes. En particulier, l'application de telles compositions sur les cheveux entraîne un toucher gras, une difficulté de rinçage. De plus, les cheveux séchés sont sans volume et ont un toucher chargé.

On connaît dans l'état de la technique des nanoémulsions comprenant une phase lipidique amphiphile constituée de phospholipides, d'un lipide cationique, d'eau et d'un filtre solaire hydrophobe.
Elles sont obtenues par un procédé d'homogénéisation à haute pression. Ces émulsions présentent l'inconvénient d'être instables au stockage aux températures traditionnelles de conservation à savoir entre 0 et 45°C. Elles conduisent à des compositions jaunes et produisent des odeurs de rance qui se développent après quelques jours de conservation. De plus, ces émulsions n'apportent pas de bonnes propriétés cosmétiques. Elles sont décrites dans la revue « DCI » d'avril 1996, pages 46-48.

Le document EP529883 décrit une composition contenant des particules microémulsifiées d'huile et un polymère aidant au dépôt et de l'eau, l'huile dans la microémulsion ayant une taille de particule inférieure à 0,14 microns. Le document EP514934 décrit une composition contenant une microémulsion de polysiloxane et un composé aidant au dépôt et de l'eau, le polysiloxane dans la microémulsion ayant une taille de particule inférieure à 0,14 microns. Ces documents ne décrivent pas de nanoémulsions comprenant l'association d'une huile, d'une silicone aminée et d'un lipide amphiphile non ionique liquide. Le document FR2718019 décrit une microémulsion contenant contenant une silicone aminée, une silicone volatile, un tensioactif et de l'eau. Ce document ne décrit pas d'huile telle que définie ci-dessous.
Le document EP-A-532256 décrit une composition contenant une microémulsion de silicone aminée, une silicone volatile, un tensioactif cationique et de l'eau. Ce document ne décrit pas d'huile telle que définie ci-dessous.

La demanderesse a découvert, de façon inattendue, de nouvelles émulsions dont les globules d'huile ont une taille moyenne inférieure à 150 nm, stables au stockage entre 0 et 45°C après au moins un mois. Les émulsions conformes à l'invention sont préparées à des températures entre 20 et 45°C et sont compatibles avec des actifs thermosensibles. Elles peuvent contenir des quantités importantes d'huile. Elles peuvent notamment contenir des quantités importantes de parfum et améliorer leur rémanence. Elles favorisent également la pénétration des actifs dans les couches superficielles de la peau et le dépôt d'actif sur les fibres kératiniques telles que les cheveux. Les cheveux traités avec ces émulsions sont lisses, brillants sans avoir un toucher ou un aspect gras, ils se démêlent facilement, sont doux et légers.

La présente invention a pour objet des émulsions huile-dans-eau comprenant une phase huileuse dispersée dans une phase aqueuse ayant des globules d'huile dont la taille moyenne est inférieure à 150 nm, caractérisées par le fait qu'elle comprennent au moins une huile, au moins une silicone aminée et une phase lipidique amphiphile laquelle comprend au moins un lipide amphiphile non-ionique liquide à une température ambiante inférieure à 45°C,
et que le rapport pondéral de la quantité de phase huileuse sur la quantité de phase lipidique amphiphile est compris entre 2 et 10, de préférence de 2 à 8 et plus particulièrement de 2 à 6, l'huile étant choisie dans le groupe formé par :
- les huiles animales ou végétales formées par des esters d'acides gras et de polyols ou bien les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone ;
- les huiles essentielles naturelles ou synthétiques ;
- les hydrocarbures ;
- les carbures halogénés ;
- les esters d'acide minéral et d'alcool ;
- les éthers et polyéthers ;
- les silicones non aminées en mélange avec au moins l'une des huiles définies ci-dessus.

Les lipides amphiphiles non-ioniques de l'invention sont préférentiellement choisis parmi les tensioactifs siliconés et les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée. On peut également utiliser les mélanges des composés ci-dessus.

Les tensioactifs siliconés utilisables selon l'invention sont des composés siliconés comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5364633 et US-A-5411744.

De préférence, le tensioactif siliconé utilisé selon la présente invention est un composé de formule (I) : 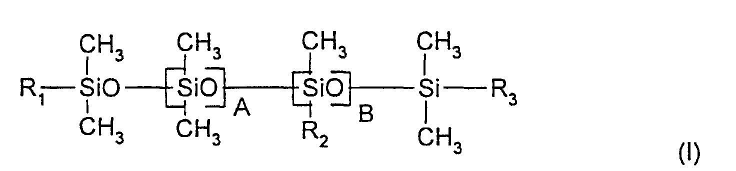 dans laquelle :
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 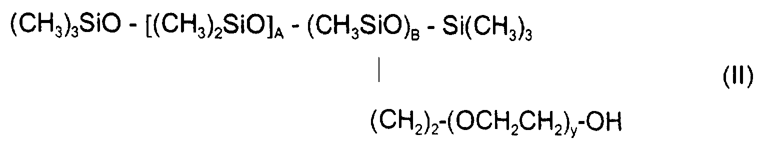 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (I), les composés de formule (III) :

HO-(CH₂CH₂O)_{y}-(CH₂)₃-[(CH₃)₂SiO]_{A'}-Si(CH₃)₂-(CH₂)₃-(OCH₂CH₂)_{y}-OH (III)

dans laquelle A' et y sont chacun indépendamment l'un de l'autre un nombre entier allant de 10 à 20.

On peut utiliser comme composés de l'invention ceux commercialisés par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est environ 22, B est environ 2 et y est environ 12 ; A est environ 103, B est environ 10 et y est environ 12 ; A est environ 27, B est environ 3 et y est environ 12.

Le composé Q4-3667 est un composé de formule (III) où A'est environ 15 et y est environ 13.

Parmi les lipides amphiphiles non ioniques, on peut plus particulièrement citer, à titre d'exemple :
- l'isostéarate de polyéthylèneglycol (ayant environ 8 unités éthylène glycol) de poids moléculaire environ 400,
- l'isostéarate de diglycéryle,
- le laurate de polyglycérol comportant environ 10 unités de glycérol,
- l'oléate de sorbitane,
- I'isostéarate de sorbitane,
- le cocoate d'α-butylglucoside ou le caprate d'α-butylglucoside.

Selon une caractéristique essentielle des compositions conformes à l'invention, les émulsions contiennent au moins une silicone aminée.

Dans tout ce qui suit ou qui précède, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements amides, les radicaux acyloxy ou acyloxyalkyls, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon l'invention, on désigne par silicone aminée toute silicone comportant au moins une amine primaire, secondaire, tertiaire ou un groupement ammonium quaternaire. On peut ainsi citer :
(a) les polysiloxanes dénommés dans le dictionnaire CTFA "amodiméthicone" et répondant à la formule : 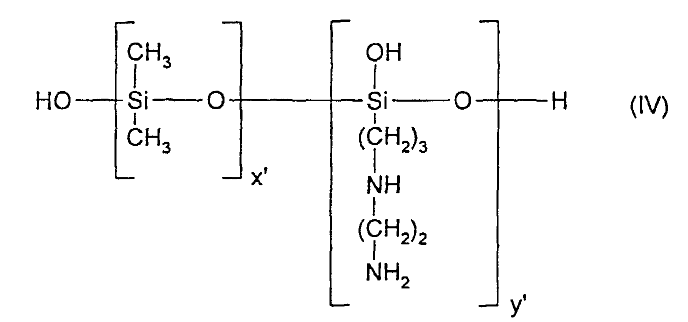 dans laquelle x' et y' sont des nombres entiers dépendant du poids moléculaire, généralement tels que ledit poids moléculaire moyen en poids est compris entre 5 000 et 500 000 environ ;
(b) les silicones aminées répondant à la formule :

   R'ₐG₃₋ₐ-Si(OSiG₂)ₙ-(OSiG_{b}R'_{2-b})ₘ-O-SiG₃₋ₐ-R'ₐ (V)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -C_{q}H_{2q}L dans laquelle q est un nombre de 2 à 8 et L est un groupement aminé éventuellement quaternisé choisi parmi les groupements :
      - NR"-CH₂-CH₂-N'(R")₂
      - N(R")₂
      - N^{⊕}(R")₃A⁻
      - NH^{⊕}(R")₂A⁻
      - NH₂^{⊕}(R")A⁻
      - N(R")-CH₂-CH₂-N^{⊕}R" H₂ A⁻,
   dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
   Un produit correspondant à cette définition est la silicone dénommée "triméthylsilylamodiméthicone", répondant à la formule : 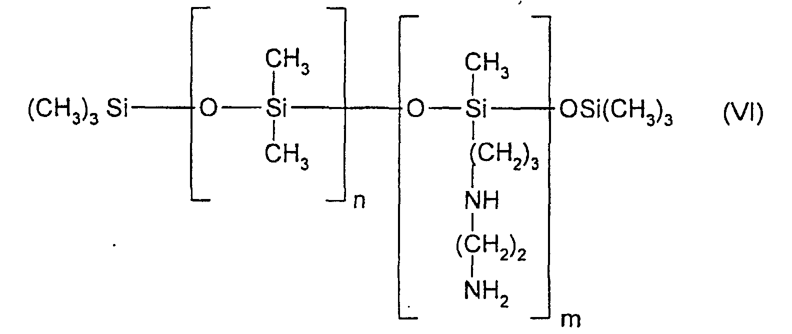 dans laquelle n et m ont les significations données ci-dessus (cf formule V).
   De tels polymères sont décrits par exemple dans la demande de brevet EP-A-95238.
(c) les silicones aminées répondant à la formule : 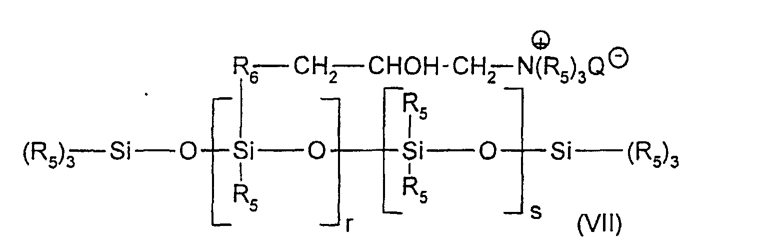 dans laquelle
   R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   Q⁻ est un anion tel qu'un ion halogénure, notamment chlorure ou un sel d'acide organique (acétate ...);
   r représente une valeur statistique moyenne de 2 à 20 et en particulier de 2 à 8;
   s représente une valeur statistique moyenne de 20 à 200 et en particulier de 20 à 50.

   De tels silicones aminées sont décrites plus particulièrement dans le brevet US 4 185 087.
   Une silicone entrant dans cette classe est la silicone commercialisée par la société Union Carbide sous la dénomination "Ucar Silicone ALE 56.
d) les silicones ammonium quaternaire de formule : 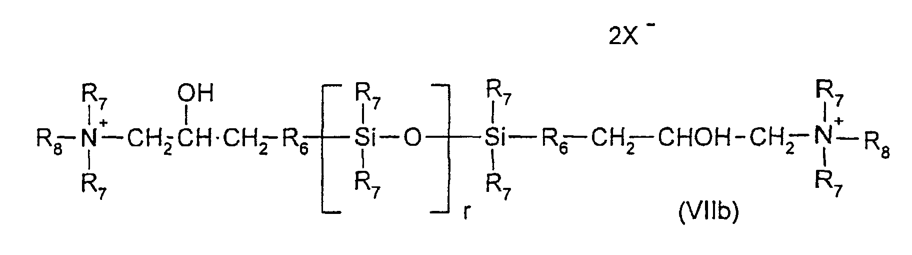 dans laquelle
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇;
   X- est un anion tel qu'un ion halogénure, notamment chlorure ou un sel d'acide organique (acétate ...);
   r représente une valeur statistique moyenne de 2 à 200 et en particulier de 5 à 100;

Ces silicones sont par exemple décrites dans la demande EP-A-0530974.

Des silicones entrant dans cette classe sont les silicones commercialisées par la société GOLDSCHMIDT sous les dénominations ABIL QUAT 3270, ABIL QUAT 3272, ABIL QUAT 3474.

Selon l'invention, les silicones aminées peuvent se présenter sous formes d'huile, de solutions aqueuses, alcooliques ou hydroalcooliques, sous forme de dispersion ou d'émulsion.
Une forme de réalisation particulièrement intéressante est leur utilisation sous forme d'émulsions en particulier sous forme de microémulsions ou de nanoémulsions.

On peut utiliser par exemple le produit commercialisé sous la dénomination "Emulsion Cationique DC 929" par la Société Dow Corning qui comprend, outre l'amodiméthicone, un agent de surface cationique dérivés des acides gras du suif dénommé Tallowtrimonium(CTFA), en association avec un agent de surface non ionique connu sous la dénomination "Nonoxynol 10".

On peut également utiliser par exemple le produit commercialisé sous la dénomination "Emulsion Cationique DC 939" par la Société Dow Corning qui comprend, outre l'amodiméthicone, un agent de surface cationique le chlorure de triméthyl cétyl ammonium en association avec un agent de surface non ionique le tridéceth-12.

Un autre produit commercial utilisable selon l'invention est le produit commercialisé sous la dénomination "Dow Corning Q2 7224" par la Société Dow Corning comportant en association le triméthylsilylamodiméthicone de formule (IV), un agent de surface non ionique de formule : C₈H₁₇-C₆H₄-(OCH₂CH₂)ₙ-OH où n = 40 dénommé encore octoxynol-40,
un autre agent de surface non ionique de formule : C₁₂H₂₅-(OCH₂-CH₂)ₙ-OH où n = 6 encore dénommé isolaureth-6, et du glycol.

Avantageusement, la silicone aminée est présente à une concentration comprise entre 0,05 et 10% en poids par rapport au poids total de l'émulsion, de préférence entre 0,1 et 5 % en poids et plus particulièrement de 0,3 à 3% en poids.

Une forme particulière d'émulsion conforme à l'invention est caractérisée par le fait que la phase lipidique amphiphile comprend en plus un ou plusieurs lipides amphiphiles ioniques.

Les lipides amphiphiles ioniques, utilisés dans les émulsions de l'invention, sont choisis dans le groupe formé par les lipides anioniques, les lipides amphotères et de préférence les lipides cationiques.

Les lipides amphiphiles anioniques sont plus particulièrement choisis dans le groupe formé par
- les sels alcalins du dicétyl- et du dimyristylphosphate ;
- les sels alcalins du cholestérol sulfate ;
- les sels alcalins du cholestérol phosphate ;
- les lipoaminoacides tels que les acylglutamates mono et disodiques ;
- les sels de sodium de l'acide phosphatidique ;
- les phospholipides ;
- les dérivés alkylsulfoniques tels que ceux de formule : 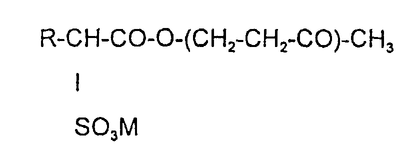
dans laquelle R représente des radicaux alkyle en C₁₆-C₂₂, en particulier les radicaux C₁₆H₃₃ et C₁₈H₃₇ pris en mélange ou séparément et M est un métal alcalin tel que le sodium.

Les lipides amphiphiles cationiques, utilisés dans les émulsions de l'invention, sont choisis, de préférence, dans le groupe formé par les sels d'ammonium quaternaire, les amines grasses et leurs sels.

Les sels d'ammonium quaternaires sont par exemple :
- ceux qui présentent la formule générale (VIII) suivante : 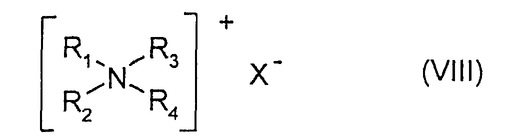 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alky(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium, comme par exemple celui de formule (IX) suivante : 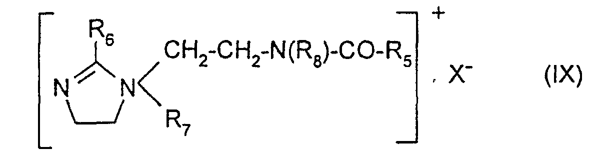 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,
- les sels de diammonium quaternaire de formule (X) : 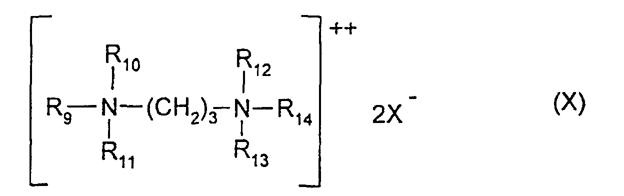 dans laquelle R₉ désigne un radical aliphatique comportant environ de 16 à 30 atomes de carbone, R₁₀, R₁₁, R₁₂, R₁₃, et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrate et méthylsulfates. De tels sels de diammonium quaternaire comprennent notamment le dichlorure de propanesuif diammonium.
- les sel d'ammonium quaternaire contenant au moins une fonction ester

Les sels d'ammonium quaternaire contenant au moins une fonction ester utilisables selon l'invention sont par exemple ceux de formule (XI) suivante : 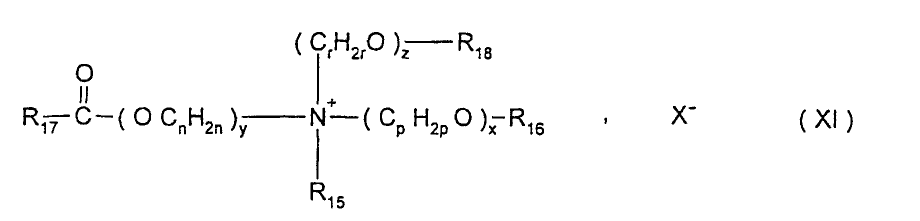 dans laquelle :
- R₁₅ est choisi parmi les radicaux alkyles en C₁-C₆ et les radicaux hydroxyalkyles ou dihydroxyalkyles en C₁-C₆;
- R₁₆ est choisi parmi :
   - le radical 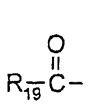
   - les radicaux R₂₀ hydrocarbonés en C₁-C₂₂ linéaires ou ramifiés, saturés ou insaturés,
   - l'atome d'hydrogène,
- R₁₈ est choisi parmi :
   - le radical 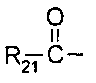
   - les radicaux R₂₂ hydrocarbonés en C₁-C₆ linéaires ou ramifiés, saturés ou insaturés,
   - l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement les sels d'ammonium de formule (XI) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 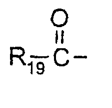
   - les radicaux méthyle, éthyle ou hydrocarbonés en C₁₄-C₂₂
   - l'atome d'hydrogène ;
- R₁₈ est choisi parmi :
   - le radical 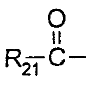
   - l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.
Avantageusement, les radicaux hydrocarbonés sont linéaires.

On peut citer par exemple les composés de formule ( XI ) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées et d'acides gras ou de mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société HENKEL, STEPANQUAT par la société STEPAN, NOXAMIUM par la société CECA, REWOQUAT WE 18 par la société REWOWITCO.

La composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30% en poids de méthylsulfate d'acyloxyéthyl dihydroxyéthyl méthyl ammonium, 45 à 60% de méthylsulfate de diacyloxyéthyl hydroxyéthyl méthyl ammonium et 15 à 30% de méthylsulfate de triacyloxyéthyl méthyl ammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VIII) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium de cétyltriméthylammonium, de benzyl diméthyl stéaryl ammonium ou encore, d'autre part, le chorure de stéaramidopropyldiméthyl (myristyl acetate) ammonium commercialisé sous la dénomination «CERAPHYL 70» par la société VAN DYK.

Selon l'invention, le chlorure de béhényltriméthylammonium est le sel d'ammonium quaternaire le plus particulièrement préféré.

Les lipides ioniques amphiphiles sont présents dans les émulsions de l'invention, de préférence, dans des concentrations allant de 0 à 60% en poids et plus particulièrement de 10 à 50 % en poids par rapport au poids total de la phase lipidique amphiphile.

Avantageusement, les lipides ioniques amphiphiles sont présents dans les émulsions de l'invention, dans des concentrations allant de 0 à 10% en poids, de préférence de 0,05 à 5 % en poids, et plus particulièrement de 0,5 à 3% en poids par rapport au poids total de l'émulsion.

Les émulsions conformes à l'invention comportent une quantité d'huile allant de préférence, de 5 à 40% en poids par rapport au poids total de l'émulsion et plus particulièrement de 8 à 30% en poids.

Les huiles pouvant être utilisées dans les émulsions de l'invention sont choisies dans le groupe formé par :
- les huiles animales ou végétales formées par des esters d'acide gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- des hydrocarbures, tels que l'hexadécane ramifié ou non ramifié et l'huile de paraffine ;
- des carbures halogénés, notamment des fluorocarbures tels que des fluoramines par exemple la perfluorotributylamine, des hydrocarbures fluorés, par exemple le perfluorodécahydronaphtalène, des fluoroesters et des fluoroethers ;
- des esters d'acide minéral et d'un alcool ;
- des éthers et des polyéthers ;
- des silicones non aminées en mélange avec au moins l'une des huiles définies ci-dessus, par exemple le décamethylcyclopentasiloxane ou le dodécaméthylcyclohexasiloxane.

Les émulsions conformes à la présente invention peuvent contenir des additifs pour améliorer, si nécessaire, la transparence de la formulation.

Ces additifs sont choisis de préférence dans le groupe formé par :
- les alcools inférieurs en C₁-C₈ tels que l'éthanol ;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.

Les additifs tels que ceux cités ci-dessus sont présents dans les émulsions de l'invention dans des concentrations allant, de préférence, de 1 à 30% en poids par rapport au poids total de l'émulsion.

En outre, l'utilisation des alcools tels que définis ci-dessus, à des concentrations supérieures ou égales à 5% en poids et de préférence supérieure à 10% en poids, permet d'obtenir des émulsions sans conservateur.

Les émulsions de l'invention peuvent contenir des actifs hydrosolubles ou liposolubles, ayant une activité cosmétique ou dermopharmaceutique. Les actifs liposolubles sont dans les globules huileux de l'émulsion, tandis que les actifs hydrosolubles sont dans la phase aqueuse de l'émulsion. On peut citer, à titre d'exemples d'actif, les vitamines telles que la vitamine E et ses dérivés, les provitamines telles que le panthénol, les humectants, les filtres solaires siliconés ou non, des agents tensioactifs, des agents conservateurs, des séquestrants, des adoucissants, des parfums, des colorants, des agents modificateurs de viscosité, des agents modificateurs de mousse, des stabilisateurs de mousse, des agents nacrants, des pigments, des agents hydratants, des agents antipelliculaires, des agents antiséborrhéiques, des protéines, des silicones, des céramides, des pseudocéramides, des acides gras à chaînes linéaires ou ramifiées en C₁₆-C₄₀ tels que l'acide méthyl-18 eicosanoique, des épaississants, des plastifiants, des hydroxyacides, des électrolytes, des polymères en particulier cationiques et des parfums.

Parmi les épaississants utilisables, on peut citer les dérivés de cellulose tels que l'hydroxyméthylpropylcellulose, les alcools gras tels que les alcools stéarylique, cétylique, béhénique, les dérivés d'algues tels que le satiagum, des gommes naturelles telles que l'adragante et des polymères synthétiques tels que les mélanges d'acides polycarboxyvinyliques commercialisés sous la dénomination CARBOPOL par la société GOODRICH et le mélange de copolymères acrylate de Na/acrylamide commercialisé sous la dénomination HOSTACERIN PN 73 par la société HOECHST.

Les globules d'huile des émulsions de l'invention, ont de préférence une taille moyenne allant de 30 à 150 nm, plus préférentiellement de 40 à 100 nm et encore plus particulièrement de 50 à 80 nm.

Les émulsions de l'invention peuvent être obtenues par un procédé, caractérisé par le fait qu'on mélange la phase aqueuse, la phase huileuse et les lipides amphiphiles, sous agitation vive, à une température ambiante inférieure à 45°C puis qu'on effectue une homogénéisation haute pression à une pression supérieure à 10⁸ Pa et de préférence allant de 12.10⁷ à 18.10⁷ Pa. Un tel procédé permet de réaliser, à température ambiante, des nanoémulsions compatibles avec des composés actifs thermosensibles, et pouvant contenir des quantités importantes d'huiles et notamment des parfums qui renferment des corps gras, sans les dénaturer.

Un autre objet de l'invention consiste en une composition à usage topique telle qu'une composition cosmétique ou dermopharmaceutique, caractérisée par le fait qu'elle est constituée par une émulsion telle que définie précédemment ou qu'elle comprend une telle émulsion. L'invention concerne plus particulièrement les compositions capillaires.

Les compositions conformes à l'invention peuvent être utilisées pour le lavage et le nettoyage des matières kératiniques telles que les cheveux et la peau.

Les compositions peuvent par exemple être utilisées pour le nettoyage ou le démaquillage de la peau.

Les compositions de l'invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage.

Les compositions peuvent être également des lotions de mise en plis, des lotions pour le brushing, des compositions de fixation (laques) et de coiffage telles que par exemple des gels, ou des mousses. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également de compositions de maquillage telles que des fonds de teint, des crèmes de jour teintées, des mascaras, des fards à joues et à paupières, des rouges à lèvres, des vernis à ongles.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

Un autre objet de l'invention est l'utilisation des émulsions telles que définies ci-dessus comme base de produits de soin et/ou de maquillage et/ou démaquillage pour la peau et/ou le visage et/ou le cuir chevelu et/ou les cheveux et/ou les ongles et/ou les cils et/ou les sourcils et/ou les muqueuses (par exemple les lèvres), tels que des lotions, des sérums, des laits, des crèmes, des eaux de toilette.

Enfin, l'invention porte également sur un procédé non-thérapeutique de soin de la peau, des cheveux, des cils, des sourcils, des ongles, des muqueuses ou du cuir chevelu, caractérisé par le fait qu'on applique sur la peau, les cheveux, les sourcils, les ongles, les muqueuses ou sur le cuir chevelu une émulsion ou une composition telle que définie ci-dessus.

Les exemples qui suivent, permettront de mieux comprendre l'invention, sans toutefois présenter un caractère limitatif.

### EXEMPLES

Pour les exemples 1 et 7, le mode opératoire suivant est mis en oeuvre : On mélange les ingrédients à l'aide d'un homogénéisateur à turbine puis on homogénéise à l'aide d'un homogénéisateur à haute pression du type Soavi-Niro à une pression d'environ 1200 bars, en plusieurs passages ( 4 à 8) en maintenant la température du produit en dessous d'environ 35°C.

### EXEMPLE 1 :

On a préparé un après-shampooing de composition suivante :
- Isostéarate de polyéthylèneglycol (8OE) commercialisé par la société UNICHEMA sous la dénomination ESTOL B UCN PEG-400 monoisostéarate BIO 4,5 g
- Chlorure de béhényltriméthylammonium (lipide amphiphile cationique) 1,6 gMA
- Huile d'avocat 15 g
- Amodiméthicone en émulsion commercialisée sous la dénomination DC2-8902 par la société DOW CORNING 1,75 gMA
- Ethanol absolu 15 g
- Glycérine 5 g
- Eau déminéralisée qsp 100 g

On obtient une émulsion dont la taille des globules d'huile est d'environ 95 nm.

Cette composition est appliquée sur des cheveux humides. Après rinçage à l'eau, les cheveux ainsi traités sont doux et lisses.

### EXEMPLE 2 :

On a préparé un après-shampooing de composition suivante :
- Isostéarate de polyéthylèneglycol (8OE) commercialisé par la société UNICHEMA sous la dénomination ESTOL B UCN PEG-400 monoisostéarate BIO 4,5 g
- Chlorure de béhényltriméthylammonium (lipide amphiphile cationique) 0,8 gMA
- Huile d'avocat 20 g
- Polydiméthylsiloxane à groupements α,ω-ammonium quaternaire commercialisé sous la dénomination ABIL QUAT 3474 par la société GOLDSCHMIDT 1,9 gMA
- Acétate de tocophérol 1 g
- Ethanol absolu 15 g
- Glycérine 5 g
- Eau déminéralisée qsp 100 g

On obtient une émulsion dont la taille des globules d'huile est d'environ 79 nm.

Cette composition est appliquée sur des cheveux humides. Les cheveux ainsi traités sont doux, lisses et faciles à démêler.

### EXEMPLE 3 :

On a préparé un après-shampooing de composition suivante :
- Isostéarate de polyéthylèneglycol (8OE) commercialisé par la société UNICHEMA sous la dénomination ESTOL B UCN PEG-400 monoisostéarate BIO 4,5 g
- Chlorure de béhényltriméthylammonium (lipide amphiphile cationique) 0,8 gMA
- Huile d'avocat 20 g
- Polydiméthylsiloxane à groupements α,ω-ammonium quaternaire commercialisé sous la dénomination ABIL QUAT 3272 par la société GOLDSCHMIDT 1 gMA
- Acétate de tocophérol 1 g
- Ethanol absolu 15 g
- Glycérine 5 g
- Eau déminéralisée qsp 100 g

On obtient une émulsion dont la taille des globules d'huile est d'environ 62 nm.

Cette composition est appliquée sur des cheveux humides. Les cheveux ainsi traités sont doux, lisses et faciles à démêler.

### EXEMPLE 4 :

On a préparé un après-shampooing de composition suivante :
- Isostéarate de polyéthylèneglycol (8OE) commercialisé par la société UNICHEMA sous la dénomination ESTOL B UCN PEG-400 monoisostéarate BIO 4,5 g
- Chlorure de béhényltriméthylammonium (lipide amphiphile cationique) 0,8 gMA
- Cire liquide de jojoba 17 g
- Polydiméthylsiloxane à groupements α,ω-ammonium quaternaire commercialisé sous la dénomination ABIL QUAT 3474 par la société GOLDSCHMIDT 1,9 gMA
- Acétate de tocophérol 1 g
- Isohexadécane 3 g
- Ethanol absolu 15 g
- Glycérine 5 g
- Eau déminéralisée qsp 100 g

On obtient une émulsion dont la taille des globules d'huile est d'environ 79 nm.

Cette composition est appliquée sur des cheveux humides. Les cheveux ainsi traités sont doux, lisses et faciles à démêler.

### EXEMPLE 5 :

On a préparé un après-shampooing de composition suivante :
- Isostéarate de polyéthylèneglycol (8OE) commercialisé par la société UNICHEMA sous la dénomination ESTOL B UCN PEG-400 monoisostéarate BIO 4,5 g
- Chlorure de béhényltriméthylammonium (lipide amphiphile cationique) 1,6 gMA
- Huile d'avocat 14 g
- Microémulsion de triméthylsilylamodiméthicone commercialisée sous la dénomination SM 2115 par la société GENERAL ELECTRIC 1,2 gMA
- Ethanol absolu 15 g
- Glycérine 5 g
- Eau déminéralisée qsp 100 g

On obtient une émulsion dont la taille des globules d'huile est d'environ 70 nm.

Cette composition est appliquée sur des cheveux humides. Les cheveux ainsi traités sont doux, lisses et faciles à démêler.

### EXEMPLE 6 :

On a préparé un après-shampooing de composition suivante :
- Isostéarate de polyéthylèneglycol (8OE) commercialisé par la société UNICHEMA sous la dénomination ESTOL B UCN PEG-400 monoisostéarate BIO 4,5 g
- Chlorure de béhényltriméthylammonium (lipide amphiphile cationique) 0,8 gMA
- Cire liquide de jojoba 5,25 g
- Huile d'avocat 5,25 g
- Polydiméthylsiloxane à groupements α,ω-ammonium quaternaire commercialisé sous la dénomination ABIL QUAT 3272 par la société GOLDSCHMIDT 1 gMA
- Cyclométhicone commercialisée par la société DOW CORNING sous la dénomination DC245 FLUID 3,5 g
- Ethanol absolu 15 g
- Glycérine 5 g
- Eau déminéralisée qsp 100 g

On obtient une émulsion dont la taille des globules d'huile est d'environ 48 nm.

Cette composition est appliquée sur des cheveux humides. Les cheveux ainsi traités sont doux, lisses et faciles à démêler.

### EXEMPLE 7 :

On a préparé un après-shampooing de composition suivante :
- Isostéarate de polyéthylèneglycol (8OE) commercialisé par la société UNICHEMA sous la dénomination ESTOL B UCN PEG-400 monoisostéarate BIO 4,5 g
- Chlorure de béhényltriméthylammonium (lipide amphiphile cationique) 1,6 gMA
- Cire liquide de jojoba 20 g
- Polydiméthylsiloxane à groupements α,ω-ammonium quaternaire commercialisé sous la dénomination ABIL QUAT 3474 par la société GOLDSCHMIDT 0,95 gMA
- Polydiméthylsiloxane à groupements α,ω-ammonium quaternaire commercialisé sous la dénomination ABIL QUAT 3272 par la société GOLDSCHMIDT 0,5 gMA
- Acétate de tocophérol 1 g
- Ethanol absolu 15 g
- Glycérine 5 g
- Eau déminéralisée qsp 100 g

On obtient une émulsion dont la taille des globules d'huile est d'environ 54 nm.

Cette composition est appliquée sur des cheveux humides. Les cheveux ainsi traités sont doux, souples et faciles à démêler.

## Revendications

1. Emulsion huile-dans-eau comprenant une phase huileuse dispersée dans une phase aqueuse dont les globules d'huile ont une taille moyenne inférieure à 150 nm, **caractérisée par le fait qu'**elle comprend au moins une huile, au moins une silicone aminée et une phase lipidique amphiphile, laquelle comprend au moins un lipide amphiphile non-ionique liquide à une température ambiante inférieure à 45°C,
et que le rapport pondéral de la quantité de phase huileuse sur la quantité de phase lipidique amphiphile est compris entre 2 et 10,
l'huile étant choisie dans le groupe formé par :
- les huiles animales ou végétales formées par des esters d'acides gras et de polyols ou bien les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone ;
- les huiles essentielles naturelles ou synthétiques ;
- les hydrocarbures ;
- les carbures halogénés ;
- les esters d'acide minéral et d'alcool ;
- les éthers et polyéthers ;
- les silicones non aminées en mélange avec au moins l'une des huiles définies ci-dessus.

2. Emulsion selon la revendication 1, **caractérisée par le fait que** le lipide amphiphile non-ionique est choisi parmi les tensioactifs siliconés et les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée, et leurs mélanges.

3. Emulsion selon la revendication 2, **caractérisée en ce que** le tensioactif siliconé est un composé de formule (I) : 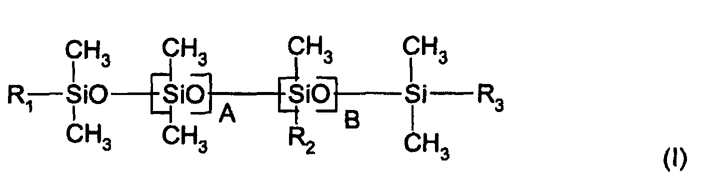 dans laquelle :
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

4. Emulsion selon la revendication 2, **caractérisée en ce que** le tensioactif siliconé est un composé de formule (II) : 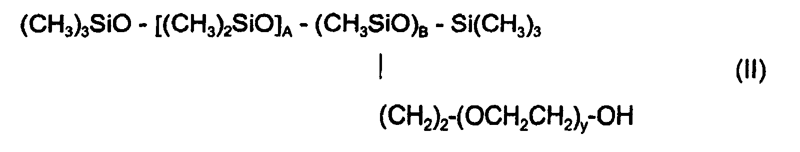 dans laquelle A est un nombre entier allant de 20 à 105, B est un nombre entier allant de 2 à 10 et y est un nombre entier allant de 10 à 20.

5. Emulsion selon la revendication 2, **caractérisée en ce que** le tensioactif siliconé est un composé de formule (III) :
HO-(CH₂CH₂O)_{y}-(CH₂)₃-[(CH₃)₂SiO]_{A'}-Si(CH₃)₂-(CH₂)₃-(OCH₂CH₂)_{y}-OH (III)
dans laquelle A' et y sont chacun indépendamment l'un de l'autre un nombre entier allant de 10 à 20.

6. Emulsion selon l'une quelconque des revendications précédentes, **caractérisée par le fait que** la silicone aminée est choisie parmi :
(a) les polysiloxanes dénommés dans le dictionnaire CTFA "amodiméthicone" et répondant à la formule : 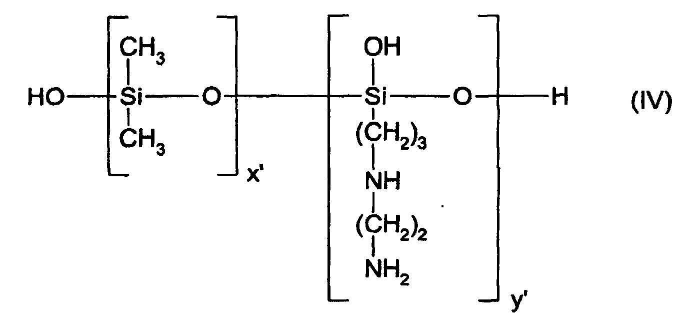 dans laquelle x' et y' sont des nombres entiers dépendant du poids moléculaire, généralement tels que ledit poids moléculaire moyen en nombre est compris entre 5 000 et 500 000 ;
(b) les silicones aminées répondant à la formule :
R'ₐG₃₋ₐ-Si(OSiG₂)ₙ-(OSiG_{b}R'_{2-b})ₘ-O-SiG₃₋ₐ-R'ₐ (V)
dans laquelle :
G est un atome d'hydrogène, ou un groupement phényle, OH, ou alkyle en C₁C₈, par exemple méthyle,
a désigne le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R' est un radical monovalent de formule -C_{q}H_{2q}L dans laquelle q est un nombre de 2 à 8 et L est un groupement aminé éventuellement quaternisé choisi parmi les groupements :
- NR"-CH₂-CH₂-N'(R")₂
- N(R")₂
- N^{⊕}(R")₃A⁻
- NH^{⊕}(R")₂A⁻
- NH₂^{⊕}(R")_{A}⁻
- N(R")-CH₂-CH₂-N^{⊕}R" H₂ A⁻,
dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
(c) les silicones aminées répondant à la formule : 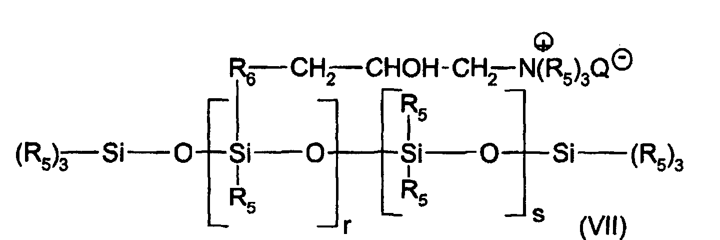 dans laquelle :
R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC ;
Q⁻ est un anion tel qu'un ion halogénure, notamment chlorure ou un sel d'acide organique (acétate ...) ;
r représente une valeur statistique moyenne de 2 à 20 et en particulier de 2 à 8;
s représente une valeur statistique moyenne de 20 à 200 et en particulier de 20 à 50.
d) les silicones ammonium quaternaire de formule : 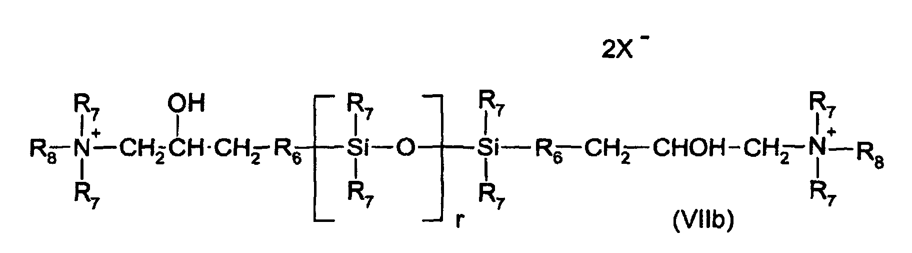 dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
X- est un anion tel qu'un ion halogénure, notamment chlorure ou un sel d'acide organique (acétate ...);
r représente une valeur statistique moyenne de 2 à 200 et en particulier de 5 à 100;

7. Emulsion selon l'une quelconque des revendications précédentes, **caractérisée en ce qu'**elle comprend, en outre, au moins un lipide amphiphile ionique.

8. Emulsion selon la revendication précédente, **caractérisée en ce que** le lipide amphiphile ionique est choisi dans le groupe formé par les lipides anioniques, les lipides amphotères, les lipides cationiques et leurs mélanges.

9. Emulsion selon la revendication 8, **caractérisée par le fait que** le lipide amphiphile cationique est choisi dans le groupe formé par les sels d'ammonium quaternaire et les amines grasses.

10. Emulsion selon la revendication 9, **caractérisée par le fait que** les sels d'ammonium quaternaire sont choisis dans le groupe formé par :
- les sels d'ammonium quaternaire de formule générale (VIII) suivante : 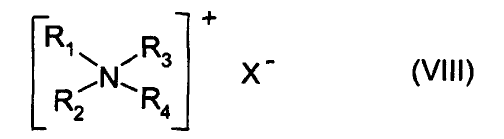 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium,
- les sels de diammonium quaternaire de formule (X) : 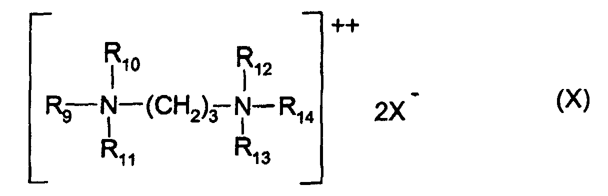 dans laquelle R₉ désigne un radical aliphatique comportant environ de 16 à 30 atomes de carbone, R₁₀, R₁₁, R₁₂, R₁₃, et R₁₄ sont choisis parmi l'hydrogène ou un radical alkyle comportant de 1 à 4 atomes de carbone, et X est un anion choisi dans le groupe des halogénures, acétates, phosphates, nitrate et méthylsulfates,
- les sel d'ammonium quaternaire contenant au moins une fonction ester.

11. Emulsion selon l'une quelconque des revendications précédentes, **caractérisée en ce que** le rapport en poids de la quantité de phase huileuse sur la phase lipidique amphiphile varie de 2 à 8 et de préférence de 2 à 6.

12. Emulsion selon l'une quelconque des revendications 1 à 11, **caractérisée par le fait que** le lipide amphiphile ionique est présent dans des concentrations allant de 0 à 60 % en poids par rapport au poids total de la phase lipidique amphiphile, et de préférence de 10 à 50% en poids.

13. Emulsion selon l'une quelconque des revendications 1 à 12, **caractérisée par le fait que** le lipide amphiphile ionique est présent dans des concentrations allant de 0 à 10% en poids, de préférence de 0,05 à 5 % en poids, et plus particulièrement de 0,5 à 3% en poids par rapport au poids total de l'émulsion.

14. Emulsion selon l'une quelconque des revendications 1 à 13, **caractérisée par le fait qu'**elle comprend une proportion d'huile allant de 5 à 40% en poids par rapport au poids total de l'émulsion.

15. Emulsion selon l'une quelconque des revendications 1 à 14, **caractérisée par le fait que** ladite silicone aminée est présente à une concentration comprise entre 0,05 et 10% en poids par rapport au poids total de l'émulsion, de préférence entre 0,1 et 5 % en poids.

16. Emulsion selon l'une quelconque des revendications 1 à 15, **caractérisée par le fait qu'**elle comprend un actif cosmétique ou dermopharmaceutique, hydrosoluble ou liposoluble.

17. Composition cosmétique à usage topique, **caractérisée par le fait qu'**elle est constituée d'une émulsion ou comprend une émulsion selon l'une quelconque des revendications 1 à 16.

18. Utilisation d'une émulsion telle que définie selon l'une quelconque des revendications 1 à 17 comme ou dans des produits de soin et/ou de lavage et/ou de maquillage et/ou démaquillage du corps et/ou du visage et/ou des muqueuses et/ou du cuir chevelu et/ou des cheveux et/ou des ongles, et/ou des cils et/ou des sourcils.

19. Procédé de traitement non-thérapeutique de la peau, des cheveux, des muqueuses, des ongles, des cils, des sourcils et/ou du cuir chevelu, **caractérisé par le fait qu'**on applique sur la peau, les cheveux, les muqueuses, les ongles, les cils, les sourcils ou le cuir chevelu une émulsion selon l'une quelconque des revendications 1 à 16 ou une composition selon la revendication 17.

20. Procédé de préparation d'une émulsion telle que définie selon l'une quelconque des revendications 1 à 16, **caractérisé par le fait qu'**on mélange la phase aqueuse et la phase huileuse et les lipides amphiphiles, sous agitation vive, à une température ambiante inférieure à 45°C puis qu'on effectue une homogénéisation haute pression à une pression supérieure à 10⁸ Pa.

21. Procédé selon la revendication 20, **caractérisé par le fait que** la pression varie de 12.10⁷ à 18.10⁷ Pa.

## Claims

1. Oil-in-water emulsion comprising an oily phase dispersed in an aqueous phase, whose oil globules have a mean size of less than 150 nm, **characterized in that** it comprises at least one oil, at least one aminated silicone and an amphiphilic lipid phase which comprises at least one nonionic amphiphilic lipid which is liquid at an ambient temperature of less than 45°C, and **in that** the weight ratio of the quantity of oily phase to the quantity of amphiphilic lipid phase is between 2 and 10,
the oil being chosen from the group formed by:
- animal or vegetable oils formed by esters of fatty acids and polyols or alternatively vegetable or animal oils of formula R₉COOR₁₀ in which R₉ represents the residue of a higher fatty acid containing from 7 to 29 carbon atoms and R₁₀ represents a linear or branched hydrocarbon chain containing from 3 to 30 carbon atoms;
- natural or synthetic essential oils;
- hydrocarbons;
- halogenated hydrocarbons;
- esters of an inorganic acid and of an alcohol;
- ethers and polyethers;
- nonaminated silicones mixed with at least one of the oils defined above.

2. Emulsion according to Claim 1, **characterized in that** the nonionic amphiphilic lipid is chosen from silicone surfactants and esters of at least one polyol chosen from the group formed by polyethylene glycol containing from 1 to 60 ethylene oxide units, sorbitan, glycerol containing from 2 to 30 ethylene oxide units, polyglycerols containing from 2 to 15 glycerol units, and; of at least one fatty acid containing at least one saturated or unsaturated, linear or branched C₈-C₂₂ alkyl chain, and mixtures thereof.

3. Emulsion according to Claim 2, **characterized in that** the silicone surfactant is a compound of formula (I) : 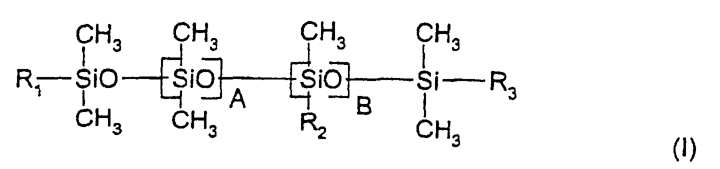 in which:
R₁, R₂ and R₃, independently of each other, represent a C₁-C₆ alkyl radical or a radical - (CH₂)ₓ-(OCH₂CH₂)_{y}-(OCH₂CH₂CH₂)_{z}-OR₄, at least one R₁, R₂ or R₃ radical not being an alkyl radical; R₄ being hydrogen, an alkyl radical or an acyl radical;
A is an integer ranging from 0 to 200;
B is an integer ranging from 0 to 50; provided that A and B are not simultaneously equal to zero;
x is an integer ranging from 1 to 6;
y is an integer ranging from 1 to 30;
z is an integer ranging from 0 to 5.

4. Emulsion according to Claim 2, **characterized in that** the silicone surfactant is a compound of formula (II) : 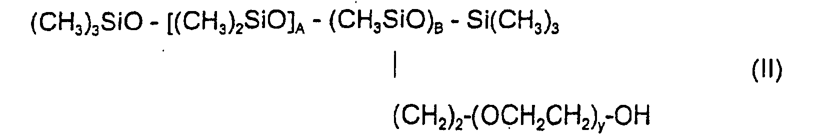 in which A is an integer ranging from 20 to 105, B is an integer ranging from 2 to 10 and y is an integer ranging from 10 to 20.

5. Emulsion according to Claim 2, **characterized in that** the silicone surfactant is a compound of formula (III) :
HO-(CH₂CH₂O)_{y}-(CH₂)₃-[(CH₃)₂SiO]_{A'} Si(CH₃)₂ (CH₂)₃-(OCH₂CH₂)_{y}-OH (III)
in which A' and y are, each independently of each other, an integer ranging from 10 to 20.

6. Emulsion according to any one of the preceding claims, **characterized in that** the aminated silicone is chosen from:
(a) the polysiloxanes called in the CTFA dictionary "amodimethicone" and corresponding to the formula: 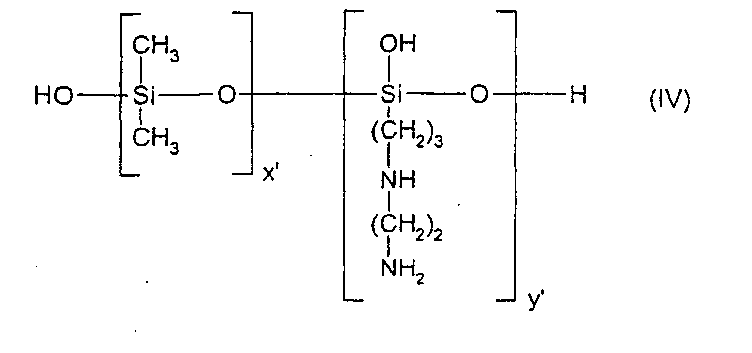 in which x' and y' are integers depending on the molecular weight, generally such that the said number-average molecular weight is between 5000 and 500,000;
(b) the aminated silicones corresponding to the formula:
R'ₐG₃₋ₐ-Si(OSiG₂)ₙ-(OSiG_{b}R'_{2-b})ₘ-O-SiG₃₋ₐ-R'ₐ (V)
in which:
G is a hydrogen atom or a phenyl, OH, or C₁-C₈ alkyl, for example methyl, group,
a designates the number 0 or an integer from 1 to 3, in particular 0,
b designates 0 or 1, and in particular 1,
m and n are numbers such that the sum (n + m) may vary in particular from 1 to 2000 and in particular from 50 to 150, it being possible for n to designate a number from 0 to 1999 and in particular from 49 to 149 and it being possible for m to designate a number from 1 to 2000, and in particular from 1 to 10;
R' is a monovalent radical of formula -C_{q}H_{2q}L in which q is a number from 2 to 8 and L is an optionally quaternized amine group chosen from the groups:
- NR"-CH₂-CH₂-N' (R")₂
- N(R")₂
- N® (R")₃A⁻
- NH^{⊕}(R")₂A⁻
- NH₂^{⊕}(R")A⁻
- N(R")-CH₂-CH₂-N^{⊕}R"H₂A⁻,
in which R" may designate hydrogen, phenyl, benzyl or a monovalent saturated hydrocarbon radical, for example an alkyl radical having from 1 to 20 carbon atoms and A⁻ represents a halide ion such as for example fluoride, chloride, bromide or iodide;
c) the aminated silicones corresponding to the formula : 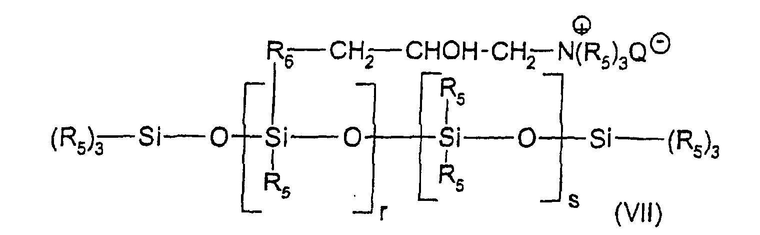 in which:
R₅ represents a monovalent hydrocarbon radical having from 1 to 18 carbon atoms, and in particular a C₁-C₁₈ alkyl radical, or a C₂-C₁₈ alkenyl radical, for example methyl;
R₆ represents a divalent hydrocarbon radical, in particular a C₁-C₁₈ alkylene radical or a divalent C₁-C₁₈ alkyleneoxy radical, for example C₁-C₈ linked to Si by an SiC bond;
Q⁻ is an anion such as a halide ion, in particular chloride or an organic acid salt (acetate and the like) ;
r represents a mean statistical value from 2 to 20, and in particular from 2 to 8;
s represents a mean statistical value from 20 to 200, and in particular from 20 to 50;
d) the quaternary ammonium silicones of formula: 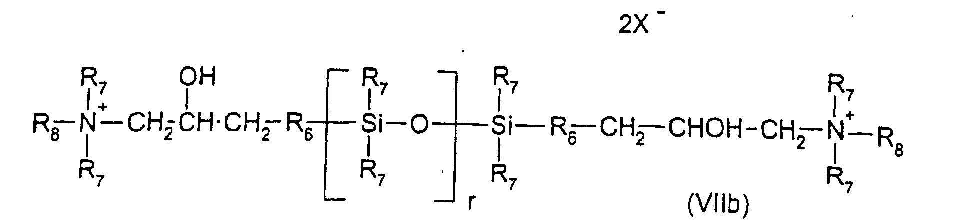 in which:
R₇, which are identical or different, represent a monovalent hydrocarbon radical having from 1 to 18 carbon atoms, and in particular a C₁-C₁₈ alkyl radical, a C₂-C₁₈ alkenyl radical or a ring comprising 5 or 6 carbon atoms, for example methyl;
R₆ represents a divalent hydrocarbon radical, in particular a C₁-C₁₈ alkylene radical or a divalent C₁-C₁₈ alkyleneoxy radical, for example C₁-C₈ linked to Si by an SiC bond;
R₈, which are identical or different, represent a hydrogen atom, a monovalent hydrocarbon radical having from 1 to 18 carbon atoms, and in particular a C₁-C₁₈ alkyl radical, a C₂-C₁₈ alkenyl radical, a radical -R₆-NHCOR₇;
X⁻ is an anion such as a halide ion, in particular chloride, or an organic acid salt (acetate and the like);
r represents a mean statistical value from 2 to 200, and in particular from 5 to 100.

7. Emulsion according to any one of the preceding claims, **characterized in that** it comprises, in addition, at least one ionic amphiphilic lipid.

8. Emulsion according to the preceding claim, **characterized in that** the ionic amphiphilic lipid is chosen from the group formed by anionic lipids, amphoteric lipids, cationic lipids and mixtures thereof.

9. Emulsion according to Claim 8, **characterized in that** the cationic amphiphilic lipid is chosen from the group formed by quaternary ammonium salts and fatty amines.

10. Emulsion according to Claim 9, **characterized in that** the quaternary ammonium salts are chosen from the group formed by:
- the quaternary ammonium salts of the following general formula (VIII): 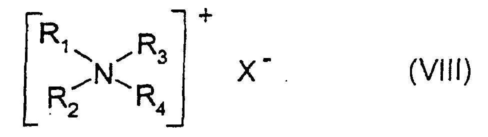 in which the radicals R₁ to R₄, which may be identical or different, represent a linear or branched aliphatic radical containing from 1 to 30 carbon atoms, or an aromatic radical such as aryl or alkylaryl. X is an anion chosen from the group comprising halides, phosphates, acetates, lactates, (C₂-C₆)alkyl sulphates and alkyl- or alkylarylsulphonates,
- the quaternary ammonium salts of imidazolinium,
- the quaternary diammonium salts of formula (X): 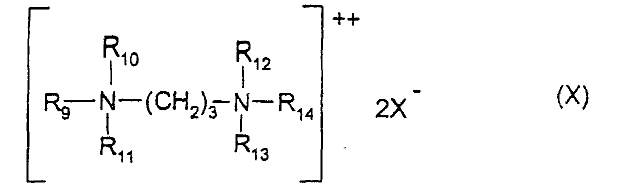 in which R₉ designates an aliphatic radical containing from about 16 to 30 carbon atoms, R₁₀, R₁₁, R₁₂, R₁₃ and R₁₄ are chosen from hydrogen or an alkyl radical containing from 1 to 4 carbon atoms, and X is an anion chosen from the group comprising the halides, acetates, phosphates, nitrate and methyl sulphates,
- the quaternary ammonium salts containing at least one ester functional group.

11. Emulsion according to any one of the preceding claims, **characterized in that** the ratio by weight of the quantity of oily phase to the amphiphilic lipid phase varies from 2 to 8 and preferably from 2 to 6.

12. Emulsion according to any one of Claims 1 to 11, **characterized in that** the ionic amphiphilic lipid is present in concentrations ranging from 0 to 60% by weight relative to the total weight of the amphiphilic lipid phase, and preferably from 10 to 50% by weight.

13. Emulsion according to any one of Claims 1 to 12, **characterized in that** the amphiphilic ionic lipid is present in concentrations ranging from 0 to 10% by weight, preferably from 0.05 to 5%. by weight, and more particularly from 0.5 to 3% by weight relative to the total weight of the emulsion.

14. Emulsion according to any one of Claims 1 to 13, **characterized in that** it comprises a proportion of oil ranging from 5 to 40% by weight relative to the total weight of the emulsion.

15. Emulsion according to any one of Claims 1 to 14, **characterized in that** the said aminated silicone is present at a concentration of between 0.05 and 10% by weight relative to the total weight of the emulsion, preferably between 0.1 and 5% by weight.

16. Emulsion according to any one of Claims 1 to 15, **characterized in that** it comprises a water-soluble or fat-soluble cosmetic or dermopharmaceutical active agent.

17. Cosmetic composition for topical use, **characterized in that** it consists of an emulsion or comprises an emulsion according to any one of Claims 1 to 16.

18. Use of an emulsion as defined according to any one of Claims 1 to 17 as or in treatment and/or washing and/or make-up and/or make-up-removing products for the body and/or the face and/or the mucous membranes and/or the scalp and/or the hair and/or the nails and/or the eyelashes and/or the eyebrows.

19. Process for the nontherapeutic treatment of the skin, the hair, the mucous membranes, the nails, the eyelashes, the eyebrows and/or the scalp, **characterized in that** an emulsion according to any one of Claims 1 to 16 or a composition according to Claim 17 is applied to the skin, the hair, the mucous membranes, the nails, the eyelashes, the eyebrows or the scalp.

20. Process for preparing an emulsion as defined according to any one of Claims 1 to 16, **characterized in that** the aqueous phase and the oily phase and the amphiphilic lipids are mixed, with vigorous stirring, at an ambient temperature of less than 45°C and **in that** a high-pressure homogenization is then carried out at a pressure greater than 10⁸ Pa.

21. Process according to Claim 20, **characterized in that** the pressure varies from 12 × 10⁷ to 18 × 10⁷ Pa.

## Patentansprüche

1. Öl-in-Wasser-Emulsion, die eine in einer wäßrigen Phase dispergierte Ölphase aufweist, wobei die Ölkügelchen eine mittlere Größe unter 150 nm aufweisen, **dadurch gekennzeichnet, daß** sie mindestens ein Öl, mindestens ein aminiertes Silicon und eine amphiphile Lipidphase enthält, die mindestens ein bei einer Umgebungstemperatur unter 45 °C flüssiges, nichtionisches, amphiphiles Lipid enthält, und dadurch, daß das Gewichtsverhältnis der Menge der Ölphase und der Menge der amphiphilen Lipidphase im Bereich von 2 bis 10 liegt, wobei das Öl ausgewählt ist unter:
- den tierischen oder pflanzlichen Ölen, die von Estern von Fettsäuren und Polyolen gebildet werden, oder den pflanzlichen oder tierischen Ölen der Formel R₉COOR₁₀, worin R₉ den Rest einer höheren Fettsäure mit 7 bis 29 Kohlenstoffatomen bedeutet und R₁₀ eine geradkettige oder verzweigte Kohlenwasserstoffkette mit 3 bis 30 Kohlenstoffatomen ist;
- den natürlichen oder synthetischen etherischen Ölen;
- den Kohlenwasserstoffen;
- den halogenierten kohlenstoffhaltigen Verbindungen;
- den Estern einer anorganischen Säure und eines Alkohols;
- den Ethern und Polyethern; und
- den nicht aminierten Siliconen im Gemisch mit mindestens einem oben definierten Öl.

2. Emulsion nach Anspruch 1, **dadurch gekennzeichnet, daß** das nichtionische amphiphile Lipid unter den siliconhaltigen grenzflächenaktiven Stoffen und den Estern mindestens eines Polyols, das unter Polyethylenglykol mit 1 bis 60 Ethylenoxideinheiten, Sorbitan, Glycerin mit 2 bis 30 Ethylenoxideinheiten und Polyglycerinen mit 2 bis 15 Glycerineinheiten ausgewählt ist, und mindestens einer Fettsäure, die mindestens eine gesättigte oder ungesättigte, geradkettige oder verzweigte C₈₋₂₂-Alkylkette enthält, und deren Gemischen ausgewählt ist.

3. Emulsion nach Anspruch 2, **dadurch gekennzeichnet, daß** der siliconhaltige grenzflächenaktive Stoff eine Verbindung der Formel (I) ist: 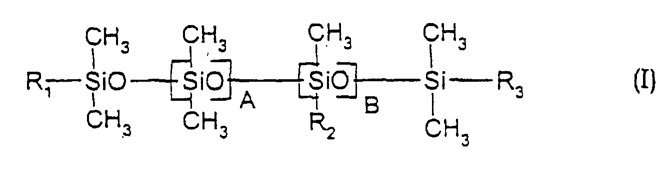 worin bedeuten:
die Gruppen R₁, R₂ und R₃ unabhängig voneinander C₁₋₆-Alkyl oder -(CH₂)ₓ - (OCH₂CH₂)_{y} - (OCH₂CH₂CH₂)_{z} - OR₄, wobei mindestens eine der Gruppen R₁, R₂ oder R₃ keine Alkylgruppe bedeutet; und wobei R₄ Wasserstoff, Alkyl oder Acyl ist;
A 0 oder eine ganze Zahl von 1 bis 200;
B 0 oder eine ganze Zahl von 1 bis 50, mit der Maßgabe, daß A und B nicht gleichzeitig Null sind;
x eine ganze Zahl von 1 bis 6;
y eine ganze Zahl von 1 bis 30;
z 0 oder eine ganze Zahl von 1 bis 5.

4. Emulsion nach Anspruch 2, **dadurch gekennzeichnet, daß** der siliconhaltige grenzflächenaktive Stoff eine Verbindung der Formel (II) ist: 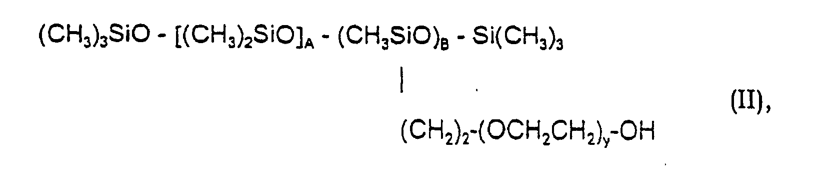 worin A eine ganze Zahl von 20 bis 105, B eine ganze Zahl von 2 bis 10 und y eine ganze Zahl von 10 bis 20 bedeutet.

5. Emulsion nach Anspruch 2, **dadurch gekennzeichnet, daß** der siliconhaltige grenzflächenaktive Stoff eine Verbindung der Formel (III) ist:
HO-(CH₂CH₂O)_{y}-(CH₂)₃-[(CH₃)₂SiO]_{A'} Si(CH₃)₂ (CH₂)₃-(OCH₂CH₂)_{y}-OH (III),
worin A' und y unabhängig voneinander eine ganze Zahl von 10 bis 20 bedeuten.

6. Emulsion nach einem der vorhergehenden Ansprüche, **dadurch gekennzeichnet, daß** das aminierte Silicon ausgewählt ist unter:
(a) den Polysiloxanen, die nach CTFA-Nomenklatur als "Amodimethicon" bezeichnet werden und der folgenden Formel entsprechen: 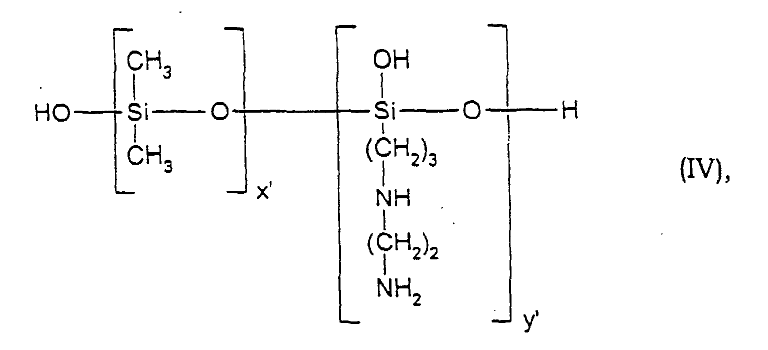 worin x' und y' ganze Zahlen sind, von denen das Molekulargewicht abhängt, wobei das Zahlenmittel des Molekulargewichts im allgemeinen im Bereich von 5000 bis 500 000 liegt;
(b) den aminierten Siliconen der Formel:
R'ₐG₃₋ₐ-Si(OSiG₂)ₙ-(OSiG_{b}R'_{2-b})ₘ-O-SiG₃₋ₐR'ₐ (V),
worin bedeuten:
G Wasserstoff, Phenyl, OH oder C₁₋₈-Alkyl, beispielsweise Methyl,
a 0 oder eine ganze Zahl von 1 bis 3 und insbesondere 0,
b 0 oder 1 und insbesondere 1,
m und n Zahlen, die so ausgewählt sind, daß die Summe (n + m) insbesondere im Bereich von 1 bis 2000 und besonders im Bereich von 50 bis 150 liegen kann, wobei n eine Zahl von 0 bis 1999 und insbesondere 49 bis 149 und m eine Zahl von 1 bis 2000 und besonders 1 bis 10 bedeuten kann;
R' eine einwertige Gruppe der Formel -C_{q}H_{2q}L, wobei q eine Zahl von 2 bis 8 und L eine gegebenenfalls quaternisierte aminierte Gruppe ist, die unter den folgenden Gruppen ausgewählt ist:
- NR"-CH₂CH₂-N'(R")₂
- N(R")2
- N^{⊕}(R")₃A⁻
- NH^{⊕}(R")₂A⁻
- NH₂^{⊕}(R")A⁻
- N(R")-CH₂-CH₂- N^{⊕}R" H₂A-,
worin R" Wasserstoff, Phenyl, Benzyl oder eine einwertige gesättigte Kohlenwasserstoffgruppe, beispielsweise eine Alkylgruppe mit 1 bis 20 Kohlenstoffatomen, und A- ein Halogenidion, beispielsweise Fluorid, Chlorid, Bromid oder Iodid, bedeuten kann.
(c) den aminierten Siliconen der folgenden Formel: 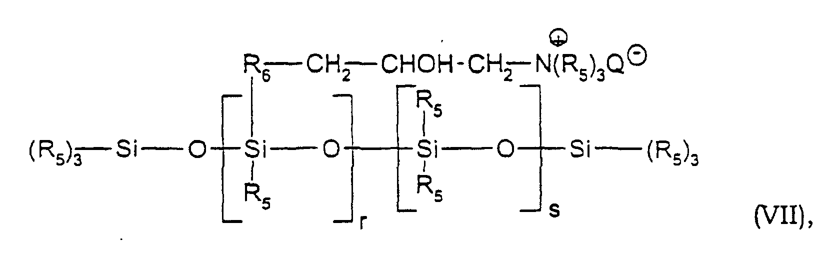 worin bedeuten:
R₅ eine einwertige Kohlenwasserstoffgruppe mit 1 bis 18 Kohlenstoffatomen und insbesondere C₁₋₁₈-Alkyl oder C₂₋₁₈-Alkenyl, beispielsweise Methyl;
R₆ eine zweiwertige Kohlenwasserstoffgruppe, insbesondere eine C₁₋₁₈-Alkylengruppe oder eine zweiwertige C₁₋₁₈-Alkylenoxygruppe, beispielsweise mit 1 bis 8 Kohlenstoffatomen, die über eine SiC-Bindung an Si gebunden ist;
Q⁻ ein Anion, wie ein Halogenidion, insbesondere Chlorid, oder das Salz einer organischen Säure (Acetat ...);
r einen statistischen Mittelwert von 2 bis 20 und insbesondere 2 bis 8;
s einen statistischen Mittelwert von 20 bis 200 und insbesondere 20 bis 50;
(d) den quartären Ammoniumsiliconen der Formel: 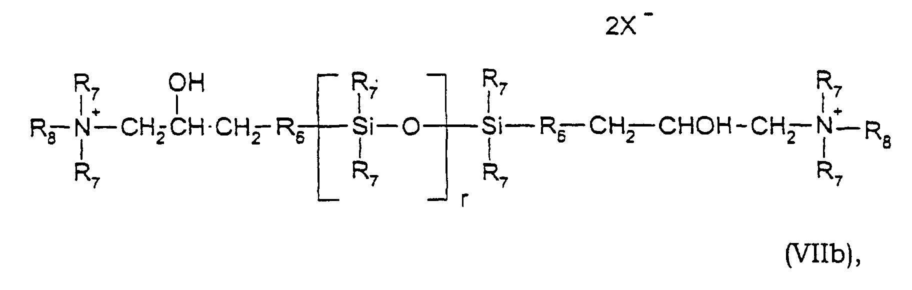 worin bedeuten:
die Gruppen R₇, die identisch oder voneinander verschieden sind, eine einwertige Kohlenwasserstoffgruppe mit 1 bis 18 kohlenstoffatomen und insbesondere C₁₋₁₈-Alkyl, C₂₋₁₈-Alkenyl oder einen Ring mit 5 oder 6 Kohlenstoffatomen, beispielsweise Methyl;
R₆ eine zweiwertige Kohlenwasserstoffgruppe, insbesondere eine C₁₋₁₈-Alkylengruppe oder eine zweiwertige C₁₋₁₈-Alkylenoxygruppe, beispielsweise mit 1 bis 8 Kohlenstoffatomen, die über eine SiC-Bindung an Si gebunden ist;
die Gruppen R₈, die identisch oder voneinander verschieden sind, Wasserstoff, eine einwertige Kohlenwasserstoffgruppe mit 1 bis 18 Kohlenstoffatomen und insbesondere C₁₋₁₈-Alkyl, C₂₋₁₈-Alkenyl, eine Gruppe -R₆-NHCOR₇;
X- ein Anion, beispielsweise ein Halogenidion, insbesondere Chlorid, oder das Salz einer organischen Säure (Acetat ...); r einen statistischen Mittelwert von 2 bis 200 und insbesondere 5 bis 100.

7. Emulsion nach einem der vorhergehenden Ansprüche, **dadurch gekennzeichnet, daß** sie ferner mindestens ein ionisches amphiphiles Lipid enthält.

8. Emulsion nach dem vorhergehenden Anspruch, **dadurch gekennzeichnet, daß** das ionische amphiphile Lipid unter den anionischen Lipiden, amphoteren Lipiden, kationischen Lipiden und deren Gemischen ausgewählt ist.

9. Emulsion nach Anspruch 8, **dadurch gekennzeichnet, daß** das kationische amphiphile Lipid unter den quartären Ammoniumsalzen und den Fettaminen ausgewählt ist.

10. Emulsion nach Anspruch 9, **dadurch gekennzeichnet, daß** die quartären Ammoniumsalze ausgewählt sind unter:
- den quartären Ammoniumsalzen der folgenden allgemeinen Formel (VIII): 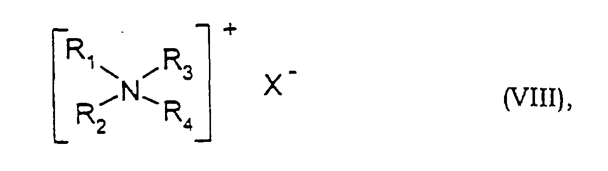
worin die Gruppen R₁ bis R₄, die identisch oder voneinander verschieden sein können, ein geradkettige oder verzweigte aliphatische Gruppe mit 1 bis 30 Kohlenstoffatomen oder eine aromatische Gruppe, wie Aryl oder Alkylaryl, bedeuten und X ein Anion ist, das unter den Halogeniden, Phosphaten, Acetaten, Lactaten, C₂₋₆-Alkylsulfaten und Alkyl- oder Alkylarylsulfonaten ausgewählt ist;
- den quartären Ammoniumsalzen von Imidazolinium;
- den quartären Diammoniumsalzen der Formel (X): 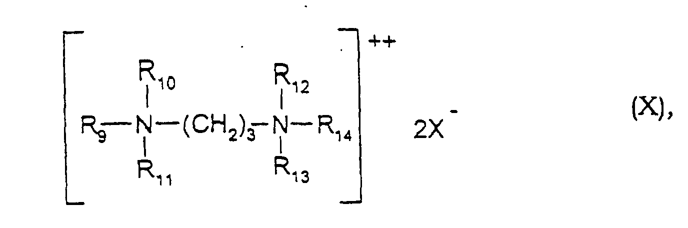 worin R₉ eine aliphatische Gruppe mit etwa 16 bis 30 Kohlenstoffatomen bedeutet, die Gruppen R₁₀, R₁₁, R₁₂, R₁₃ und R₁₄ unter Wasserstoff oder den Alkylgruppen mit 1 bis 4 Kohlenstoffatomen ausgewählt sind und X ein Anion ist, das unter den Halogeniden, Acetaten, Phosphaten, Nitrat und Methylsulfaten ausgewählt ist; und
- den quartären Ammoniumsalzen, die mindestens eine Estergruppe enthalten.

11. Emulsion nach einem der vorhergehenden Ansprüche, **dadurch gekennzeichnet, daß** das Gewichtsverhältnis der Menge der Ölphase und der Menge der amphiphilen Lipidphase im Bereich von 2 bis 8 und vorzugsweise 2 bis 6 liegt.

12. Emulsion nach einem der Ansprüche 1 bis 11, **dadurch gekennzeichnet, daß** das ionische amphiphile Lipid in Konzentrationen von 0 bis 60 Gew.-%, bezogen auf das Gesamtgewicht der amphiphilen Lipidphase, und vorzugsweise 10 bis 50 Gew.-% vorliegt.

13. Emulsion nach einem der Ansprüche 1 bis 12, **dadurch gekennzeichnet, daß** das ionische amphiphile Lipid in Konzentrationen von 0 bis 10 Gew.-%, vorzugsweise 0,05 bis 5 Gew.-% und insbesondere 0,5 bis 3 Gew.-%, bezogen auf das Gesamtgewicht der Emulsion, vorliegt.

14. Emulsion nach einem der Ansprüche 1 bis 13, **dadurch gekennzeichnet, daß** sie einen Ölanteil von 5 bis 40 Gew.-%, bezogen auf das Gesamtgewicht der Emulsion, enthält.

15. Emulsion nach einem der Ansprüche 1 bis 14, **dadurch gekennzeichnet, daß** das aminierte Silicon in einer Konzentration von 0,05 bis 10 Gew.-%, bezogen auf das Gesamtgewicht der Emulsion, und vorzugsweise 0,1 bis 5 Gew.-% vorliegt.

16. Emulsion nach einem der Ansprüche 1 bis 15, **dadurch gekennzeichnet, daß** sie einen wasserlöslichen oder fettlöslichen kosmetischen oder dermopharmazeutischen Wirkstoff enthält.

17. Kosmetische Zusammensetzung zur topischen Anwendung, **dadurch gekennzeichnet, daß** sie aus einer Emulison nach einem der Ansprüche 1 bis 16 besteht oder eine solche Emulsion enthält.

18. Verwendung einer Emulsion nach einem der Ansprüche 1 bis 17 als Produkt zur Pflege und/oder zum Waschen und/oder zum Schminken und/oder zum Abschminken des Körpers und/oder des Gesichts und/oder der Schleimhäute und/oder der Kopfhaut und/oder der Haare und/oder der Nägel und/oder der Wimpern und/oder der Augenbrauen oder in solchen Produkten.

19. Verfahren zur nicht therapeutischen Behandlung der Haut, der Haare, der Schleimhäute, der Nägel, der Wimpern, der Augenbrauen und/oder der Kopfhaut, **dadurch gekennzeichnet, daß** auf die Haut, die Haare, die Schleimhäute, die Nägel, die Wimpern, die Augenbrauen oder die Kopfhaut eine Emulsion nach einem der Ansprüche 1 bis 16 oder eine Zusammensetzung nach Anspruch 17 aufgetragen wird.

20. Verfahren zur Herstellung einer Emulsion nach einem der Ansprüche 1 bis 16, **dadurch gekennzeichnet, daß** die wäßrige Phase, die Ölphase und die amphiphilen Lipide unter kräftigem Rühren bei einer Umgebungstemperatur unter 45 °C vermischt werden und anschließend eine Hochdruckhomogenisierung bei einem Druck über 10⁸ Pa durchgeführt wird.

21. Verfahren nach Anspruch 20, **dadurch gekennzeichnet, daß** der Druck im Bereich von 12 · 10⁷ bis 18 · 10⁷ Pa liegt.
